# EUROPEAN PATENT SPECIFICATION

(11) **EP 3 442 607 B1**
(45) Date of publication and mention of the grant of the patent: **03.06.2020**
(21) Application number: 17720333.8
(22) Date of filing: 11.04.2017
(51) Int. Cl.: A61L 15/58

(54) **SILICONE FREE PRODUCTS WITH EQUIVALENT ADHESION AND SILICONE-LIKE LOW TRAUMA SKIN CONTACT PROPERTIES**
SILIKONFREIE PRODUKTE MIT ÄQUIVALENTER HAFTUNG UND SILIKONÄHNLICHEN HAUTSCHONENDEN KONTAKTEIGENSCHAFTEN
PRODUITS SANS SILICONE AYANT UNE ADHÉRENCE ÉQUIVALENTE ET DES PROPRIÉTÉS DE CONTACT AVEC LA PEAU FAIBLEMENT TRAUMATISANT SIMILAIRE À UNE SILICONE

(30) Priority: 11.04.2016 US 201662320781 P
(43) Date of publication of application: 20.02.2019
(73) Proprietor: Lohmann Corporation, Hebron, KY 41048 (US)
(72) Inventor: CARR, Jan, Charlottesville VA 22911 (US); SCHUETTE, Mario, 56179 Vallendar (DE); SHORT, Austin, Euclid OH 44123 (US); HOFFMANN, Gregg, Culpeper VA 22701 (US)
(74) Representative: Samson & Partner Patentanwälte mbB
(86) International application number: PCT/US2017/026965
(87) International publication number: WO 2017/180595

(56) References cited:
- EP-A1- 1 060 719
- WO-A1-2011/120507
- WO-A1-2014/168726
- WO-A1-2015/124669
- WO-A2-2012/027377
- US-A- 5 810 756
- DATABASE WPI Week 200778 Thomson Scientific, London, GB; AN 2007-835547 XP002771923, & JP 2007 209515 A (NICHIBAN KK) 23 August 2007 (2007-08-23)
- Alan Neil: "Innovative Non-Silicone Low Trauma Adhesives versus Traditional Silicone Technology A Review and Comparison", , 1 January 2016 (2016-01-01), XP055389643, Retrieved from the Internet: URL:http://www.softstickadhesive.com/docum ents/lohmann-wp-web.pdf [retrieved on 2017-07-11]
- Gozde Karabiyik: "Nonsilicone Adhesives for Low-Trauma Skin Bonding", MEDICAL DEVICE & DIAGNOSTIC INDUSTRY, 1 February 2013 (2013-02-01), XP055389709, Retrieved from the Internet: URL:http://www.adhesivesresearch.com/wp-co ntent/uploads/2013/08/MDDI_Nonsilicone-Adh esives-for-Low-Trauma-Skin-Bonding.pdf [retrieved on 2017-07-11]

## Description

### RELATED APPLICATIONS

This application claims the benefit of priority to U.S. Provisional Application No. 62/320,781, titled "Silicone Free Products with Equivalent Adhesion and Silicone-like Low Trauma Skin Contact Properties" filed April 11, 2016.

### FIELD OF INVENTION

Silicone-free products with advanced skin properties for low trauma applications. These include: modulus matched silicone-free products with advanced skin properties for low trauma applications; perforated high MVTR silicone-free gel products with advanced skin properties for low trauma applications; hybrid silicone-free gel adhesive coatings with advanced skin contact properties for low trauma applications; compounded hybrid silicone-free gel adhesives with advanced skin contact properties for low trauma applications.

### BACKGROUND

A medical pressure sensitive adhesive material, such as a tape, has a structure that a pressure sensitive adhesive layer is formed on at least one side of a base material. Since the medical pressure sensitive adhesive tape is intended to be applied to skin, and often applied on various skin types, it is designed after due consideration so as not to cause pain on removal for example, during dressing changes and to minimize skin irritation.

Specifically, as a base material of the medical pressure sensitive adhesive tape, a backing or fabric, nonwoven fabric, plastic film, paper or the like that is skin contouring with good flexibility and gas permeability is generally used. In order to prevent moisture generated by perspiration retention, a base material made of a material having excellent moisture permeability is also preferred. Further, a tape where the base material and tape components have good elastic extention and stretchability so as to permit conforming to the movement of human skin are described.

As a pressure sensitive adhesive for the medical pressure sensitive adhesive tape, a pressure sensitive adhesive such as a synthetic rubber, standard solvent acrylic pressure sensitive adhesive or high temperature acrylate, which minimizes trauma to skin during removal and repositioning of product reducing marked skin irritation, is used. In order to minimize pain and skin stripping by the adhesive tape, components constituting the pressure sensitive adhesive product are used which facilitate the transfer of the forces of removal over the surface of the components of the adhesive tape product rather than the skin of the wearer. The re-distribution of this energy results in less trauma to the skin and a product that produces no pain on removal.

The adhesive strength of the medical pressure sensitive adhesive in such products is required to be sufficiently high enough to secure absorbent cotton, gauze and bandages, as well as catheters, tubing, and devices. On the other hand, the adhesive strength of the skin contacting medical pressure sensitive adhesive tape has to be developed so that the majority of skin cells are not disrupted during peeling and removal of the pressure sensitive adhesive tape.

As described above, the medical pressure sensitive adhesive product is designed with the base material, components of the pressure sensitive adhesive product, and adhesion level of the pressure sensitive tape, etc. in such a manner that the product provides an adequate balance of adhesion to secure the product to the skin while minimizing trauma to the skin during wear and removal. A balance of such properties is difficult to achieve and the majority of conventional medical pressure sensitive adhesives, tapes and products have been developed to secure the product while the need for atraumatic removal either remains insufficient or goes unmet.

From the viewpoint of skin irritation by a medical pressure adhesive tape, a medical pressure sensitive adhesive tape is often applied over a longer period of time or repeatedly on a specific site (repeat exposure). In such a case, when skin cells are removed and the outer layers of the skin are repeatedly stripped the skin can be predisposed to irritation, mechanical injury and ultimately breakdown. In order to prevent the possibility of such an effect, it is necessary to reduce the chances of minor irritation of the skin as much as possible. This requirement has largely been ignored during the development of conventional medical pressure sensitive tapes and products due to the complexity of the challenge.

Silicone products have wide acceptance and lead the market as the "go to" products in advanced wound care applications where no pain on removal is a major requirement. Silicone has been instrumental in helping to establish the primary low trauma performance requirements as pain on removal from skin, comfort during wear, and ability to reposition.

Other than silicone, current low trauma technologies and competitive approaches to this end include: hydrocolloids, hydrogels, polyurethanes and standard acrylic PSA's. Of the gel technologies, hydrocolloids provide absorption and are typically used on wet skin as in ostomy applications.

Hydrogels provide gel character and soft skin adhesion and due to a high concentration of water, can be cooling to skin. Polyurethanes provide good gel character and soft removal from skin but can offer some processing challenges. Standard pressure sensitive acrylics that are softer than adhesives can be coated at higher coat weights to provide some gel characteristics, but the tack strength is typically a lot higher than a silicone, making the overall feel of the adhesive although somewhat soft, still quite different from a silicone.

WO2011120507 and WO2014168726 disclose medical silicone-free adhesive devices, comprising pressure adhesive adhesives.

### BRIEF SUMMARY

The scope of the invention is defined by the claims. Any references in the description to methods of treatment refer to the compositions and devices of the present invention for use in a method for treatment of the human (or animal) body by therapy.

The present invention describes a medical pressure sensitive adhesive tape comprising a pressure sensitive adhesive material layer formed on at least one side of a release liner, wherein the adhesive material layer comprises compounded polymeric hybrid adhesives combining two discrete gel adhesive layers, and wherein the pressure sensitive adhesive material is silicone-free.

This technology relates to silicone-free gel adhesive products that match the feel and performance properties of silicone adhesive products for advanced wound care applications. These gel adhesive products are designed to meet the needs and expectations of end users in the silicone market for features such as lack of pain on removal, for providing comfort during wear on the body and having the ability to reposition once the product has been applied to the body. These new gel adhesive products provide the benefits of silicone products while improving on the major disadvantages of silicone adhesive products such as being more cost -effective and being gamma sterilizable. The adhesives for this technology may be solvent acrylic, hot melt acrylics or modified block rubbers.

In addition, this technology relates to body bandages and skin wraps for standard wound care, surgery, ostomy, skin tears and ulcers - to include bandages, foam wound dressings, non-adherent layers, NPWT (negative pressure wound therapy) drapes and components, incise and surgical drapes, excision transfer layers, scar compression sheets and wound closure strips for low trauma applications where performance equivalence to silicone products is required in preventing pain on removal, providing comfort during wear, and the ability to reposition. The medical pressure sensitive adhesive tape strength to bakelite panel is within a range of about 0.2 to 20.0 N/24 mm and also a range of about 0.6 to 15.0 N/24 mm. The adhesive strength to human skin at an inner lower arm is within a range of about 0.05 to 10.0 N/24 mm and also about 0.1 to 8.0 N/24 mm. The adhesive strength to stainless steel is within a range of about 0.05 to 15.0 N/24 mm and also about 0.1 to 10.0 N/24 mm. The adhesive strength to an inner lower arm on sensitive human skin is about 0.05 to about 5.0 N/24mm, while the adhesive strength to human skin without sensitivities is about 0.05 to about 10.0 N/24mm or about 5.0 to about 15.0N/24mm when higher adhesion or device fixation is a concern. Conversely, the same adhesive tape with a high density perforation pattern (ties approximately 3mm), the adhesive tape strength is within a range of about 0.01 to about 6.0N/24mm, while the same adhesive tape with a lower density perforation pattern, the tape strength is within a range of about 0.05 to about 12.0 N/24mm.

### BRIEF DESCRIPTION OF DRAWINGS

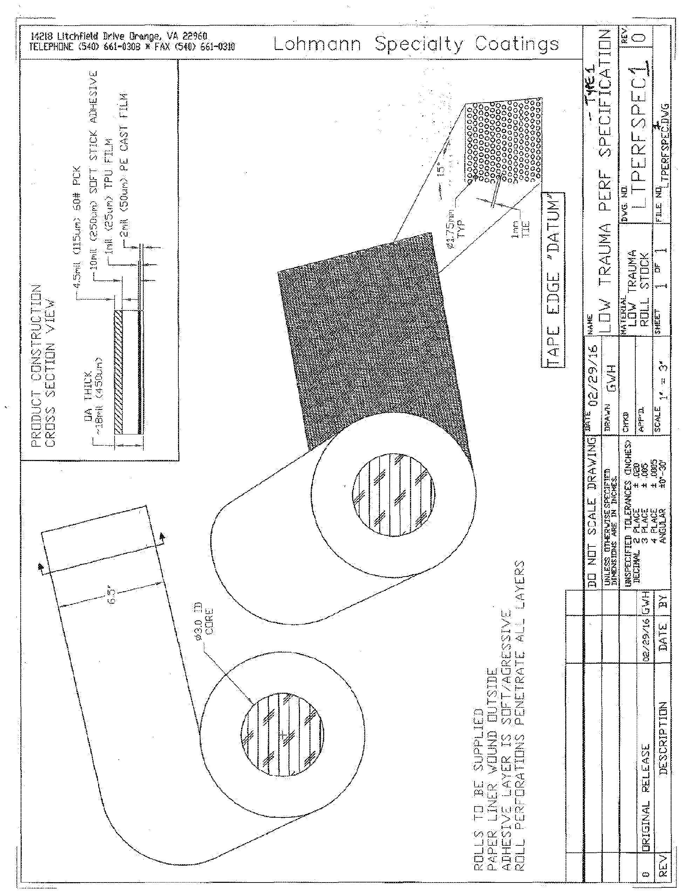

**Figure 1****:** Product cross-section view, low trauma perforated roll perforation pattern 1.

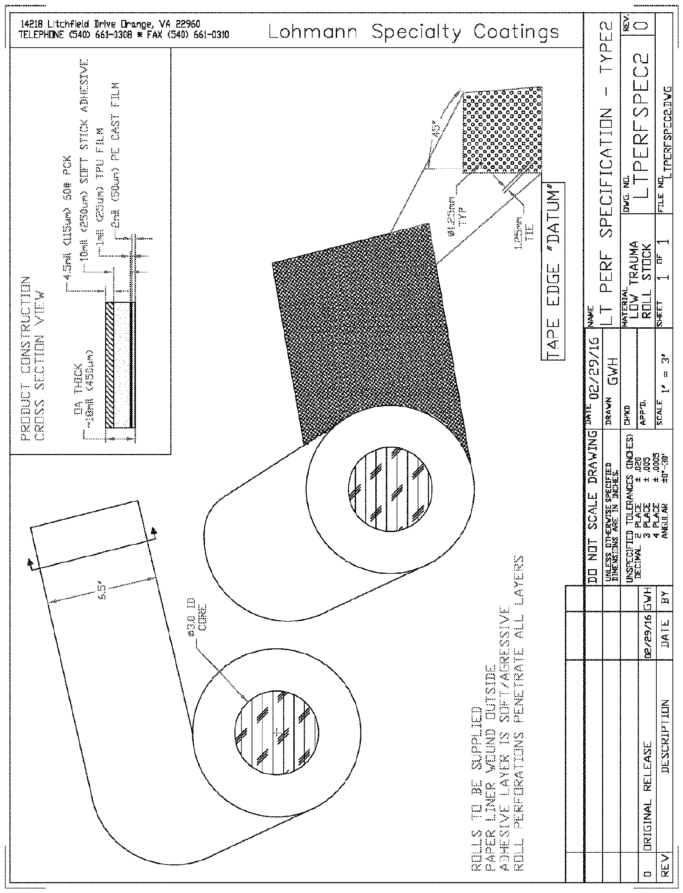

**Figure 2****:** Product cross-section view, low trauma perforated roll, perforation pattern 2.

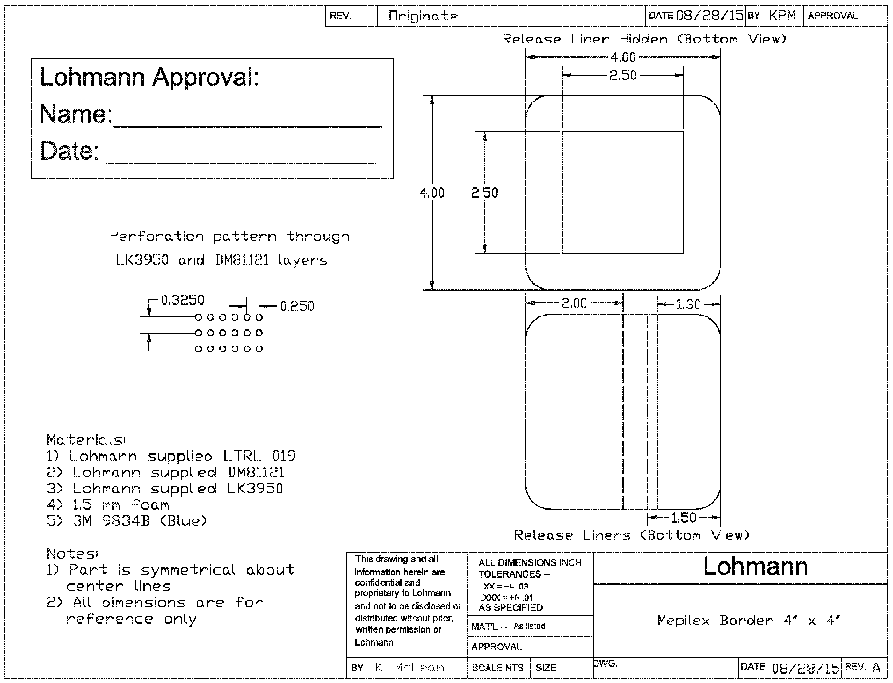

**Figure 3****:** Silicone-free Foam Border Dressing product with perforation layer.

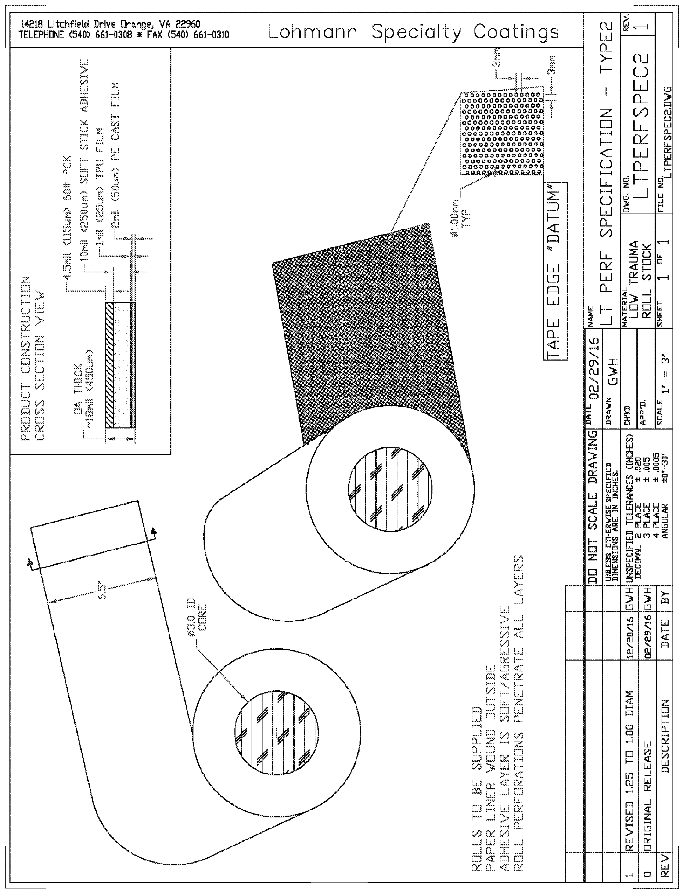

**Figure 4****:** Product cross-section view, low trauma perforated roll, perforation pattern 3.

### DETAILED DESCRIPTION

Described is the use of hybrid polymeric coatings to produce cost-effective silicone-free products having optimal gel character and the performance and feel of silicone adhesive products. Also described is the use of compounded polymeric hybrid adhesives to produce cost-effective silicone-free products with optimal gel character and the performance and feel of silicone adhesive products.

The gel adhesive products that are commercially available in the medical market to address atraumatic removal of wound products are not considered direct replacements for silicone and are distinguishable by both feel of the adhesive and performance limitations of these constructions. Conversely, the modulus of these new gel products has been uniquely tailored to match the modulus of a broad variety of skin types, making these gel adhesive products suitable as direct replacements for silicone gel products involving skin contact. Bench testing shows that perforations are an integral feature of products used in advanced wound care applications and these patterns largely contribute to the function of the product. The perforated patterns of these gel adhesive products was developed to provide the percentage open area and MVTR (moisture vapor transmission rate) for equivalent or much improved function and wear performance vs. current silicone products currently in the market.

These new gel adhesive products match the needs of various skin types in the same way as silicone adhesives and are much more cost-effective, positioning them viable options for wound care and applications where silicone products have previously been cost-restrictive or the only option of choice.

The modulus range for skin types and a modulus range of optimized gel adhesive products have been identified, where ideal skin contact properties are achieved between the wound care product and skin in order to improve feel and performance of the product during wear and removal. Optimizing the properties in a wound care product to include skin contact properties allows the extension and contraction of skin interfacing with the product to not only reduce mechanical stress on the skin, but to minimize irritation and promote overall comfort.

In Examples 5 & 6 the modulus matching effect is achieved by combining bulk adhesive properties of discrete layer 2 gel adhesive and skin contact properties of discrete layer 1 adhesive in various thickness ratios. Since bulk properties of adhesives are very different, when the bulk layer is combined with another discrete skin contacting layer at different thicknesses many versatile combinations and matched moduli are possible. With this method, the overall modulus of the adhesive is controlled not only by the adhesive type used as the bulk layer but also the ratio of the bulk adhesive to the skin contacting layer. Matching the modulus of discrete adhesive layers in this way provides the ability to offer both skin contact properties and bulk adhesive properties that are unique and many varied combinations for different skin types. An example of a preferred modulus would be 1:1, where 76.2 µm [3 mils] of a non-silicone adhesive gel is combined with 76.2 µm [3 mils] of a particular bulk adhesive, or another preferred modulus would be 101.6 µm [4 mils] of a non-silicone adhesive gel is combined with 152.4 µm [6 mils] of yet a different bulk adhesive. In another preferred modulus, 76.2 µm [3 mils] of a non-silicone adhesive gel is combined with 177.8 µm [7 mils] of yet a different bulk adhesive. (Table 1) Data provided in Tables 2A-2C show how the choice of adhesive type (standard acrylic PSA's) and choice of carrier substrate (polyurethane film, polyurethane foam, polyethylene film and polyester nonwoven) can influence the bonding strength of an adhesive and wear times of a product. Table 2A shows the influence of substrate (polyester nonwoven, polyurethane foam, polyurethane film, PE film) on a product's wear time (# days of wear on skin before failure) when the adhesive is constant and it also shows the influence of adhesive on wear duration (# days of wear) when the substrate is constant. Table 2B shows 4 different nonwoven substrates and a single adhesive option (gel adhesive). Table 2C shows a single nonwoven type with 4 different adhesives.

In one embodiment of the non-silicone gel development, the modulus of carrier components is taken into consideration and is correlated with the modulus of skin types to create an overall synergistic product where performance equivalence to silicone products such as no pain on removal and comfort during wear is required. These gel adhesive products offer end users not only select adhesion levels but products that are tailored to particular skin properties providing high performance, little to no pain on removal with silicone-free constructions for skin contact applications.

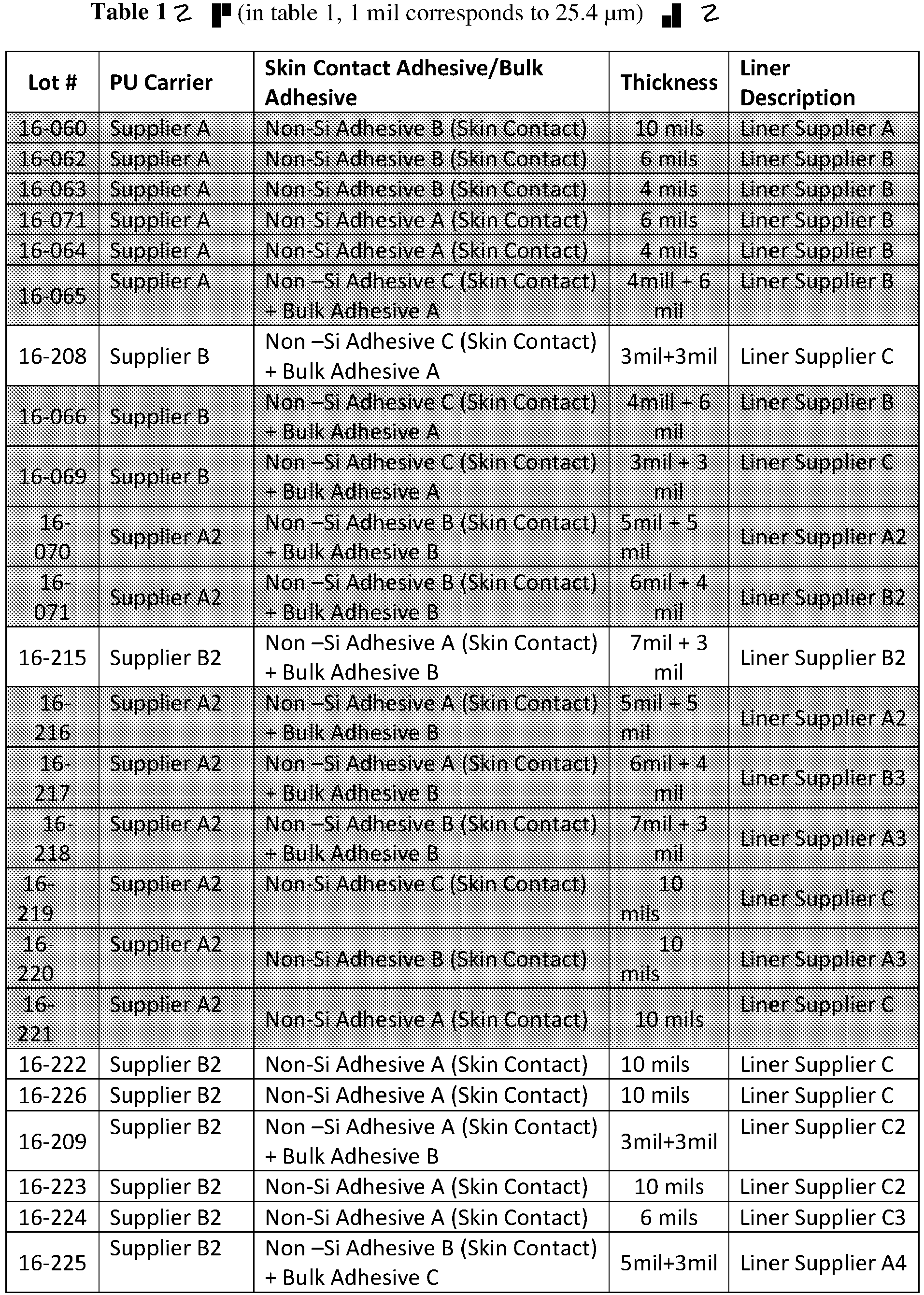

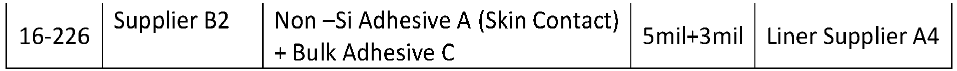

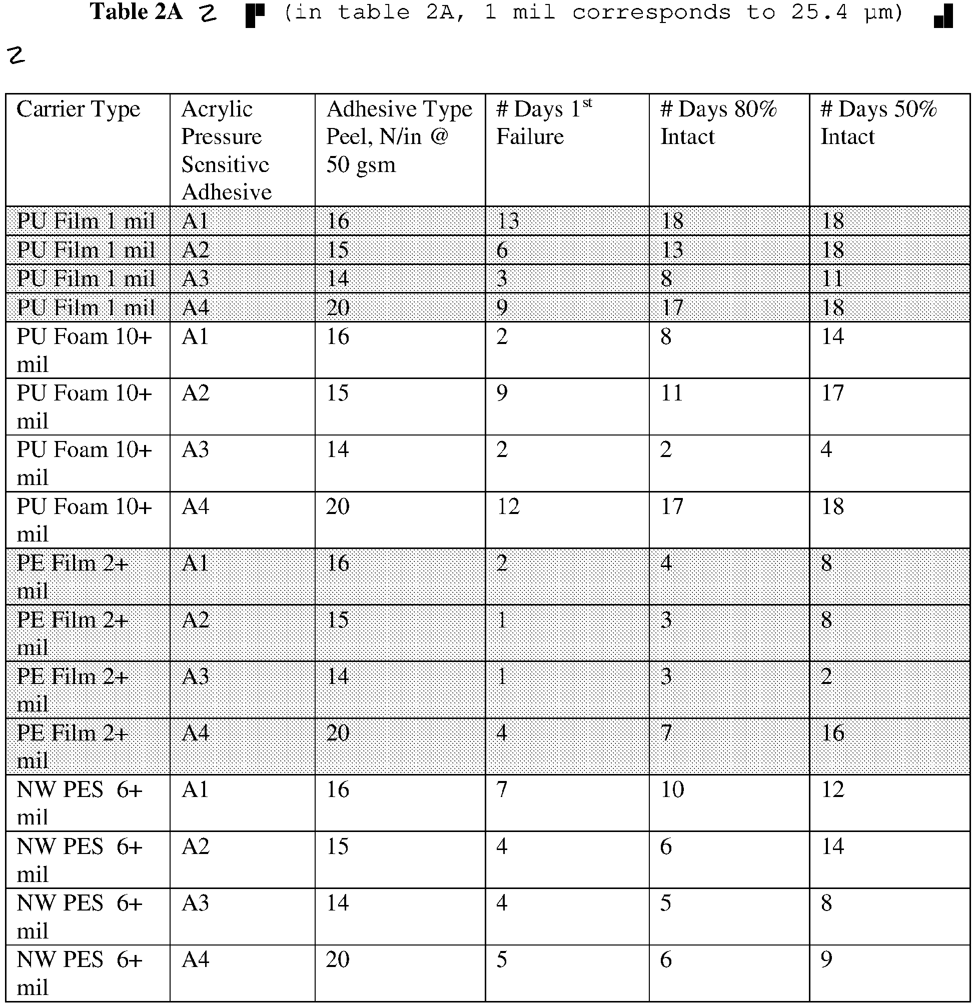

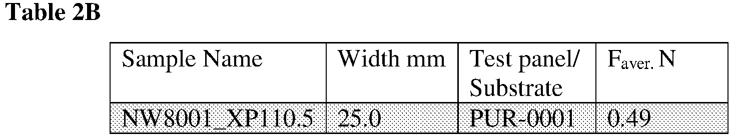

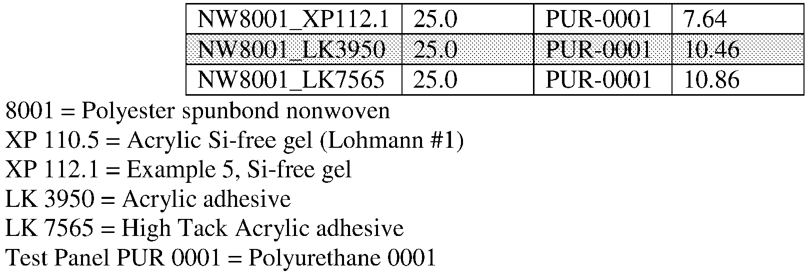

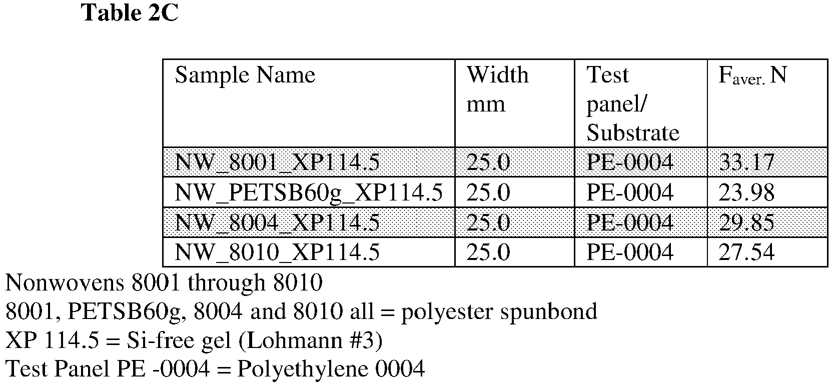

Qualitative assessments of the low trauma effects of the non-silicone gel adhesives conducted in direct comparisons against market leading silicon adhesive products by experienced wound care practitioners indicate the non-silicone gel adhesives are comparable to, or preferred over market leading silicone products. This feedback is based on the ability to reposition these adhesives after initial bonding to the skin, comfort during contact with the skin and lack of pain on removal of the adhesive. (Table 3).

**Table 3**

| | Most Comfortable | No Pain on Removal | Repositionability |
|---|---|---|---|
| Silicone Mkt Leader #1 | 25% | 15% | 7% |
| Silicone Mkt Leader #2 | 12% | 30% | 14% |
| Acrylic Si-free gel (Lohmann #1) | 33% | 30% | 54% |
| Modified rubber Si-free gel (Lohmann #2) | 17% | 20% | 7% |
| Modified rubber Si-free gel (Lohmann #3) | 13% | 5% | 18% |

### EXAMPLES

The following examples are non-narrowing, illustrative examples of the technology. In all examples, the adhesives used are either solvent acrylic, hot melt acrylics or modified block rubbers. Where 'gel adhesive' is indicated, the preference in these examples is either hot melt acrylic or modified rubbers.

### Example 1: (not according to the invention)

The gel layer is either a solvent acrylic, a hot melt acrylate or synthetic rubber based adhesive such as SBS, SIS, or SES. The preferred carrier is a polyurethane film but can be a nonwoven, PET, PE, PP, foam, etc. The PU carrier film has a casting sheet for reinforcement and better handling. The PU used in these examples is inert but could contain an active and is a next generation idea. The thickness of the gel adhesives tend to be > 100 gsm without an upper limit. However, 100-300 gsm is the range for the gel character and skin properties that are targeted as optimal. Properties with less gel character (closer to standard PSA) although still relevant, are less pertinent in these examples; thicknesses for these adhesives are optimally in the 30 - 75 gsm range. The release liner for the gel adhesive can be a standard 'easy' release, controlled release or an ultra-easy release (custom formulation) on the closed (skin) or open sides depending on requirements.

| |
|---|
| 60# PCK Paper = 4.5 mils |
| NS Gel Adhesive = 10 mils |
| TPU film carrier =1 mil |
| PE casting sheet = 2 mils (**may or mav not be included) |
| Total =Approx. 18 mils |

### Example 2: Modulus matched (not according to the invention)

Modulus matched - Modulus of the gel adhesive, modulus of the carrier and modulus of the backing are purposely matched to produce a dressing with optimal skin contact interface and value-add wear properties such as the ability to reposition and remove wrinkles while the dressing is kept in place, ease of removal of the dressing from skin, repositionability of the dressing once applied, conformability & contouring of the layers to skin over time and improved comfort during wear. The gel layer is either a solvent acrylic, a hot melt acrylate or synthetic rubber based adhesive such as SBS, SIS, SES, etc.

| |
|---|
| 60# PCK Paper = 4.5 mils |
| NS Gel Adhesive = 10 mils |
| TPU film carrier =1 mil |
| PE casting sheet = 2 mils (**may or mav not be included) |
| Total =Approx. 18 mils |

### Example 3: High MTVR (not according to the invention)

High MVTR is indicated in this example since surface area of the gel adhesive layer is greatly reduced once perforated and the vapor transmission of the adhesive increases significantly. In this example the perforation pattern and the transmission rate are approximated close to a commercial product in order to demonstrate equivalent % open area. The gel layer is either a solvent acrylic, a hot melt acrylate or synthetic rubber based adhesive such as SBS, SIS, SES, etc. Perforations in the converted dressing example are approximately 1.75 mm with 1 mm tie.

| |
|---|
| 60# PCK Paper = 4.5 mils |
| NS Gel Adhesive =10 mils |
| TPU film carrier = 1 mil |
| Foam Island = 60 or 137 mils |
| Solvent Acrylic = 1.5 mils |
| TPU film backing =1 mil |
| Total = Approx. 80 - 160 mils |

### Example 4: Hybrid adhesive (not according to the invention)

This is a compound of either (2) acrylic hot melt adhesives or a compound of (2) rubber based adhesives. The gel adhesive in this example is an adhesive purchased from a supplier that is compounded with another adhesive from this or another supplier to achieve unique properties.

The gel layer is either a solvent acrylic, a hot melt acrylate or synthetic rubber based adhesive such as SBS, SIS, SES, etc. PU is the preferred carrier/backing.

| |
|---|
| 60# PCK Paper = 4.5 mils |
| NS Gel Adhesive = 10 mils |
| TPU film carrier = 1 mil |
| PE casting sheet = 2 mils (**may or mav not be included) |
| Total =Approx. 18 mils |

### Example 5: Hybrid Coating

Gel A is an acrylic hot melt PSA and Gel B is a different acrylic hot melt. The chemical composition of gel adhesive B is an acrylic hot melt. The gel adhesive layers A and B are discrete layers with different thicknesses. In Example 5, Gel A is 100 gsm and Gel B is 150 gsm. The carrier in Example 5 is PU with no tissue between adhesive layers and it may contain an active.

| |
|---|
| 60# PCK Paper = 4.5 mils |
| NS Gel Adhesive =10 mils |
| TPU film carrier = 1 mil |
| PE casting sheet = 2 mils (**may or may not be included) |
| Total =Approx. 18 mils |

### Example 6: Hybrid Transfer Coating

Hybrid Transfer coating is an adhesive (layers A+B) that can be removed from the release liner (as 1 layer) and applied to a carrier of choice. Hybrid coating is a compounded adhesive (same or 2 different suppliers) that is coated as a single layer. With Example 6, PET or another release liner is used to facilitate the transfer of the hybrid adhesive coating during lamination to another carrier of choice. The chemical composition of gel adhesive A is an acrylic hot melt PSA or synthetic rubber adhesive. The chemical composition of gel adhesive B is an acrylic hot melt PSA or synthetic rubber adhesive.

| |
|---|
| 60# PCK Paper = 4.5 mils |
| NS Gel Adhesive =10 mils |
| TPU film carrier = 1 mil |
| PE casting sheet = 2 mils (**may or may not be included) |
| Total =Approx. 18 mils |

Example 1: Silicone free tape/pad (not according to the invention)

| | |
|---|---|
| 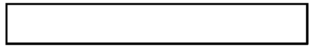 | Release liner |
| 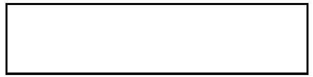 | Gel |
| 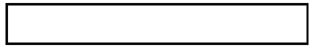 | Backing/Carrier |
| 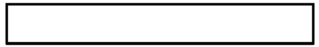 | Casting Sheet |

Example 2: Modulus matched tape/pad (not according to the invention)

| | |
|---|---|
| 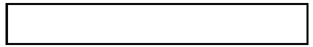 | Release liner |
| 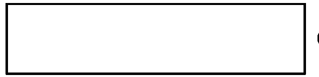 | Gel adhesive |
| 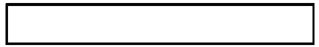 | Backing/Carrier |

Example 3: High MVTR tape/pad (not according to the invention)

| | |
|---|---|
| 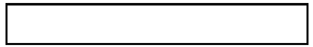 | Release liner |
| 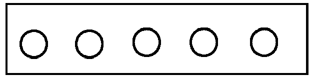 | Perforated gel adhesive on PU film carrier |
| 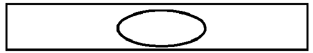 | Foam + adhesive coated PU |

| | |
|---|---|
| *Tri-laminate layer = gel adhesive coated polyurethane film is perforated and laminated to a single sided adhesive coated polyurethane film + foam island | |

Example 4: Hybrid adhesive tape/pad (not according to the invention)

| | |
|---|---|
| 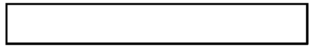 | Release liner |
| 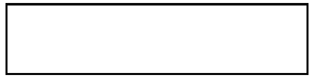 | Gel adhesive |
| 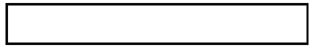 | Backing/Carrier |
| 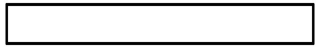 | Casting sheet |

Example 5: Hybrid coating tape/pad

| | |
|---|---|
| 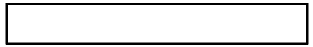 | Release liner |
| 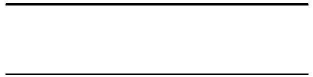 | Gel adhesive A |
| 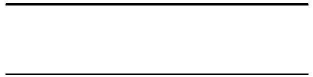 | Gel adhesive B |
| 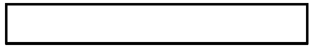 | Backing |

Example 6: Hybrid transfer coating tape/pad

| | |
|---|---|
| 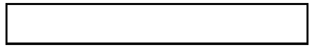 | Release liner |
| 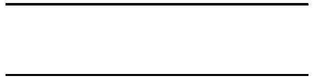 | Gel adhesive A |
| 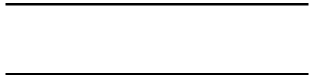 | Gel adhesive B |
| 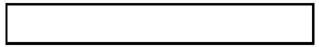 | PET release liner |

## Claims

1. A medical pressure sensitive adhesive tape comprising a pressure sensitive adhesive material layer formed on at least one side of a release liner, wherein the adhesive material layer comprises compounded polymeric hybrid adhesives combining two discrete gel adhesive layers, and wherein the pressure sensitive adhesive material is silicone-free.

2. The medical pressure sensitive adhesive tape of claim 1 wherein the tape is perforated.

3. The medical pressure sensitive adhesive tape of claim 1, wherein the pressure sensitive adhesive is a rubber pressure sensitive adhesive or an acrylate pressure sensitive adhesive.

4. The medical pressure sensitive adhesive tape of claim 1, wherein the adhesive strength to bakelite panel is within a range of about 0.6 to 15.0 N/24 mm.

5. The medical pressure sensitive adhesive tape according to claim 1, wherein the adhesive strength to human skin at an inner lower arm is within a range of about 0.1 to 8.0 N/24 mm.

6. The medical pressure sensitive adhesive tape according to claim 1, wherein the adhesive strength to stainless steel is within a range of about 0.1 to 10.0 N/24 mm.

7. The medical pressure sensitive adhesive tape according to claim 4, wherein the pressure sensitive adhesive is an acrylate pressure sensitive adhesive.

8. The medical pressure sensitive adhesive tape of claim 1, comprising a base material which is a fabric, a nonwoven fabric, a film, a perforated film or a composite material thereof.

9. The medical pressure sensitive adhesive tape of claim 1, comprising a hybrid transfer tape which is not on a fixed carrier.

## Patentansprüche

1. Ein medizinisches druckempfindliches Klebeband, umfassend eine druckempfindliche Klebematerialschicht, welche auf mindestens einer Seite einer Trennschicht ausgebildet ist, wobei die Klebematerialschicht vermische polymere Hybridkleber umfasst, welche zwei diskrete Gelklebematerialschichten kombinieren und wobei das druckempfindliche Klebematerial silikonfrei ist.

2. Das medizinische druckempfindliche Klebeband nach Anspruch 1, wobei das Band perforiert ist.

3. Das medizinische druckempfindliche Klebeband nach Anspruch 1, wobei der druckempfindliche Kleber ein druckempfindlicher Gummikleber oder ein druckempfindlicher Akrylatkleber ist.

4. Das medizinische druckempfindliche Klebeband nach Anspruch 1, wobei die Klebefestigkeit an Bakelitplatte im Bereich von etwa 0,6 bis 15,0 N/24 mm ist.

5. Das medizinische druckempfindliche Klebeband nach Anspruch 1, wobei die Klebefestigkeit an menschliche Haut an einer Unterarminnenseite im Bereich von etwa 0,1 bis 8,0 N/24 mm ist.

6. Das medizinische druckempfindliche Klebeband nach Anspruch 1, wobei die Klebefestigkeit an Edelstahl im Bereich von etwa 0,1 bis 10,0 N/24 mm ist.

7. Das medizinische druckempfindliche Klebeband nach Anspruch 4, wobei der druckempfindliche Kleber ein druckempfindlicher Akrylatkleberl ist.

8. Das medizinische druckempfindliche Klebeband nach Anspruch 1, umfassend ein Grundmaterial welches ein Stoff, ein Vliesstoff, ein Film, ein perforierter Film oder ein Verbundmaterial daraus ist.

9. Das medizinische druckempfindliche Klebeband nach Anspruch 1, umfassend eine Hybridtrennschicht, welche nicht auf einem festen Träger angeordnet ist.

## Revendications

1. Bande adhésive sensible à la pression à usage médical comprenant une couche de matériau adhésif sensible à la pression formée sur au moins un côté d'une doublure antiadhésive, dans laquelle la couche de matériau adhésif comprend des adhésifs hybrides polymères combinés combinant deux couches discrètes d'adhésif en gel, et dans laquelle le matériau adhésif sensible à la pression est exempt de silicone.

2. Bande adhésive sensible à la pression à usage médical selon la revendication 1, laquelle bande est perforée.

3. Bande adhésive sensible à la pression à usage médical selon la revendication 1, dans laquelle l'adhésif sensible à la pression est un adhésif sensible à la pression en caoutchouc ou un adhésif sensible à la pression en acrylate.

4. Bande adhésive sensible à la pression à usage médical selon la revendication 1, dans laquelle la force adhésive à un panneau de bakélite est située dans la plage allant d'environ 0,6 à 15,0 N/24 mm.

5. Bande adhésive sensible à la pression à usage médical selon la revendication 1, dans laquelle la force adhésive à la peau humaine au niveau de l'intérieur de l'avant-bras est située dans la plage allant d'environ 0,1 à 8,0 N/24 mm.

6. Bande adhésive sensible à la pression à usage médical selon la revendication 1, dans laquelle la force adhésive à l'acier inoxydable est située dans la plage allant d'environ 0,1 à 10,0 N/24 mm.

7. Bande adhésive sensible à la pression à usage médical selon la revendication 4, dans laquelle l'adhésif sensible à la pression est un adhésif sensible à la pression en acrylate.

8. Bande adhésive sensible à la pression à usage médical selon la revendication 1, comprenant un matériau de base qui est une étoffe, une étoffe non tissée, un film, un film perforé ou un matériau composite de ceux-ci.

9. Bande adhésive sensible à la pression à usage médical selon la revendication 1, comprenant une bande de transfert hybride qui n'est pas sur un support fixe.
